Europäisches Patentamt

European Patent Office

Office européen des brevets

Publication number: **0 296 325**
**A2**

# EUROPEAN PATENT APPLICATION

Application number: **88106056.0**

Int. Cl.⁴ **A61F 13/00 , A61F 13/12**

Date of filing: **13.11.85**

Priority: **21.11.84 DK 5528/84**

Date of publication of application:
**28.12.88 Bulletin 88/52**

Publication number of the earlier application in accordance with Art.76 EPC: **0 185 197**

Designated Contracting States:
**DE FR GB IT NL SE**

Applicant: **Mölnlycke AB**

**S-405 03 Göteborg(SE)**

Applicant: **Tytex A/S**
**Industrivej 21**
**DK-7430 Ikast(DK)**

Inventor: **Kristensen, Johannes Nyvang**
**Laesogade 3**
**DK-7430 Ikast(DK)**
Inventor: **Thygesen, Eskild Georg**
**Broholmvej 14 Tulstrup**
**DK-7430 Ikast(DK)**
Inventor: **Grindsted, Erik**
**Frederiksberg Alle 19A 4. th**
**DK-1820 Frederiksberg C(DK)**
Inventor: **Jundell, Malte**
**Bokvägen 8**
**S-435 00 Mölnlycke(SE)**

Representative: **Patentanwälte Leinweber & Zimmermann**
**Rosental 7/II Aufg.**
**D-8000 München 2(DE)**

## A fixation bandage for holding a dressing in position on the head of a person.

A fixation bandage made from elastic material and adapted to hold a dressing such as a compress or the like firmly in a position on the head of a person, is mainly formed as a hose in one piece preferably by knitting or crocheting.

The hose comprises a holding portion (7') adapted to receive the head of a person, a fastening portion (5') and at least one gap (3'). The fastening portion (5') forms a cuff, a collar or the like, which is intended to embrace the neck of the person.

The fixation bandage is so shaped and has an elasticity such that both the holding portion (7') and the fastening portion (5') can be slipped over the head in such a manner as to have its fastening portion placed around the neck.

By means of the flexible and elastic fixation bandage thus provided the firm holding of the dressing in position is ensured, even in the case of extreme movements of the head. Moreover the fixation bandage is easy to apply whether this is done by the injured person or by a person assisting him in the application of the fixation bandage.

FIG. 1

# A FIXATION BANDAGE FOR HOLDING A DRESSING IN POSITION ON THE HEAD OF A PERSON

The present invention relates to a fixation bandage consisting of elastic material and intended to hold a wound dressing, such as a compress, firmly in position on a peripheral body portion of a person, said fixation bandage being preferably made by knitting or crocheting and being shaped mainly as a hose or said hose or flexible tube comprising a holding portion for receiving the body portion considered, at least one fastening portion and at least one gap, said fastening portion forming a cuff, collar, loop or the like for embracing a joint adjacent to the body portion in question of a person, the fixation bandage being so shaped and having an elasticity such that both the holding portion and the fastening portion can be slipped over the body portion considered in such a manner as to have its fastening portion placed around the adjacent joint.

A fixation bandage of this type is disclosed in US-A-3 279 465. This prior fixation bandage is adapted to be placed on those parts of the human body, like the trunk and the abdomen. The bandage has the shape of a T-shirt and is made of a knitted fabric comprising inelastic yarns and elastic yarns which are arranged in a zig-zag configuration.

It is known to hold wound dressings in position by means of conventional bandages, particularly gauze bandages. However, this has the disadvantage that the correct holding of the wound dressing depends very much on the tightness with which the bandage has been applied so that there is a risk that the holding of the wound dressing may become either too loose or too tight.

It is also known to hold wound dressings in position by means of elastic tubular meshed fabrics, which are intended to provide an improved holding of the dressing because they can expand and contract in all direction. Thereby the above mentioned drawback in respect of tightness of the bandage is eliminated. However, these tubular meshed fabrics cannot be used in connection with all types of dressings.

The application of dressings to the head, e.g. ear dressings, has hitherto been particularly troublesome and time consuming. Such head dressings are fastened by means of a gauze bandage which must be wound around the whole of the head of the patient. The consumption of material for the application of such a bandage is very considerable, and it requires great skill to apply a head bandage in such a manner that it will remain firmly in position.

It is the object of the present invention to remedy the drawbacks mentioned above by providing a fixation bandage which is flexible and elastic and ensures the maintenance in position of the dressing on the head even in the case of extreme movements thereof, and which is easy to apply, whether it is done by the injured person himself or by a person assisting him for the purpose.

To achieve this object the invention is **characterized in** that the hose is closed at one end in the holding portion, said end being adapted to be applied to the top of the head, and is open at its other end in the fastening portion, that the gap is provided in the side of the hose and extends mainly in the transverse direction of the hose and is located adjacent the open end of the hose, and that the fastening portion forms a collar which is adapted, after both portions of the hose have been slipped over the head of the patient, to be placed around the neck of the patient, whereby the gap will form a free opening for at least part of the face of the patient.

Further developments of the invention are set forth in the depending claims.

The invention will now be described in more detail with reference to an exemplifying, non-limiting embodiment thereof illustrated in the accompanying drawings, in which

Fig. 1 shows a fixation bandage adapted to be placed on the head of a person,

Figs 2 and 3 are two illustrations of the fixation bandage of Fig. 1 as applied to a patient.

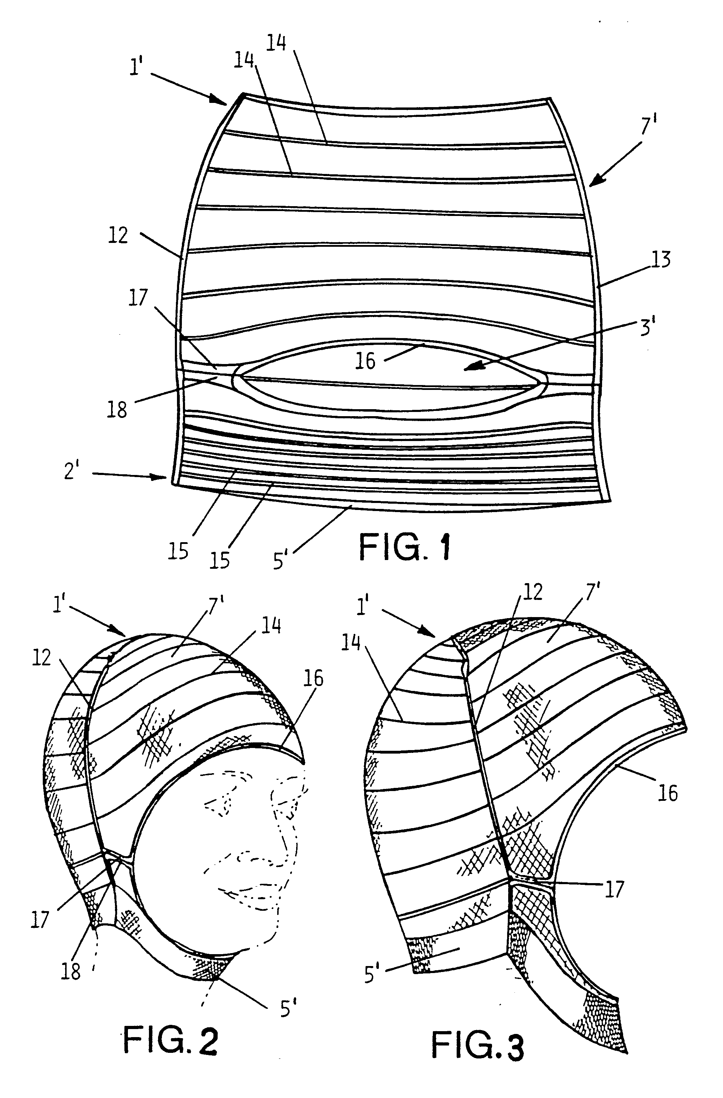

Fig. 1 shows a fixation bandage which is adapted to be placed on the head of a patient to hold a dressing in position.

The fixation bandage is made from two pieces knitted from elastic material and interconnected along the marginal edges 12, 13. The hose-like unit thereby formed is closed at one end 1' and open at its other end 2'. The fixation bandage is provided with a gap 3' extending in the transverse direction of the hose and adapted to form a free opening for the face of the user.

The holding portion 7' is made with an open knitting and is narrowed towards its closed end for adaptation to the shape of the top of the head. Transversely of the holding portion 7' re-inforcing threads having a stronger elasticity than the other threads of the holding portion are incorporated at regular intervals.

In a portion adjacent the open end 2' of the hose, the knitting is tight, and elastic re-inforcement threads 15 are provided tightly as compared with the threads 14 of the holding portion. This end portion at the open end of the hose is adapted to function as a collar 5' around the neck of the user.

The gap 3' is borded by an elastic thread 16 adapted to hold the edge of the gap in position around the face of the user. At each side of the gap a relatively strong elastic 17, 18 is arranged, and the edges of the hose are made with a certain elastic stretchability, but relatively stiff.

Figs. 2 and 3 show the fixation bandage of Fig. 1 as applied to a user. As is clearly apparent from Figs. 2 and 3, the fixation bandage for a head dressing illustrated in these figures has an excellent fit and can simply be pulled over the head without any auxiliary means. The holding portion 7′ placed above the top of the head is held firmly in position by means of the fastening portion 5′ formed as a collar.

The elastic thread 16 around the gap, the elastic members 17, 18 and the edges 12, 13 cooperate to hold the holding portion and the marginal elastic thread 16 in position.

The illustrated embodiment of a fixation bandage for a head dressing is particularly suitable for the holding of compresses and the like applied to the top of the head and or over the ears.

The shape of the gap and the elastic properties of the holding portion can, however, be varied for adaptation to other uses, e.g. for holding a dressing on the eyes or on the forehead in position.

## Claims

1. A fixation bandage consisting of elastic material and intended to hold a wound dressing, such as a compress, firmly in position on a peripheral body portion of a person, said fixation bandage being preferably made by knitting or crocheting and being shaped mainly as a hose or flexible tube in one piece, said hose or flexible tube comprising a holding portion (7) for receiving the body portion considered, at least one fastening portion (5) and at least one gap (3), said fastening portion forming a cuff, collar, loop or the like for embracing a joint adjacent to the body portion in question of a person, the fixation bandage being so shaped and having an elasticity such that both the holding portion (7) and the fastening portion (5) can be slipped over the body portion considered in such a manner as to have its fastening portion placed around the adjacent joint, **characterized in** that the hose is closed at one end (1′) in the holding portion, said end being adapted to be applied to the top of the head, and is open at its other end (2′) in the fastening portion (5′), that the gap (3′) is provided in the side of the hose and extends mainly in the transverse direction of the hose and is located adjacent the open end (2′) of the hose, and that the fastening portion (5′) forms a collar which is adapted, after both portions of the hose have been slipped over the head of the patient, to be placed around the neck of the patient, whereby the gap (3′) will form a free opening for at least part of the face of the patient.

2. A fixation bandage as in claim 1, **characterized in** that the hose is knitted or crocheted, and the collar (5′) is made with stronger elasticity than the remainder of the hose, and that this elasticity may, if desired, be strengthened by the incorporation in the web of threads (14) running in the transverse direction of the hose and having a stronger elasticity than the other elastic threads in the knitting or crocheting pattern.

3. A fixation bandage as in claim 1 or 2, **characterized in** that the closed end portion of the hose is narrowed for adaptation to the shape of the top of the head.

4. A fixation bandage as in any of the beforegoing claims, **characterized in** that the hose is made with different types of knitting (a-b) distributed over the length of the hose.

5. A fixation bandage as in any of the beforegoing claims, **characterized in** that the hose is provided with a colour code (11) to indicate size.

6. A fixation bandage as in any of the beforegoing claims, **characterized in** that the hose is made from two knitted or crocheted webs which are interconnected at their edges (12, 13) to form the hose.

FIG. 1

FIG. 2

FIG. 3